# EUROPEAN PATENT APPLICATION

(11) **EP 4 148 146 A1**
(43) Date of publication of application: **15.03.2023**
(21) Application number: 21196366.5
(22) Date of filing: 13.09.2021
(51) Int. Cl.: C12Q 1/6869, C12Q 1/6886, C07K 14/47, G01N 33/68

(54) **METHOD TO GENERATE PERSONALIZED NEOANTIGENS OF A TUMOR OF A PATIENT**

(71) Applicant: OncoDNA, 6041 Charleroi (BE)
(72) Inventor: Detiffe, Jean-Pol, 6500 Beaumont (BE); Van Huffel, Christophe, 1332 Rixensart (BE)
(74) Representative: Calysta NV

(57) **Abstract**

A process for generating a plurality of neoantigens from a sample obtained from a patient comprising the steps of: sequencing the DNA and/or the RNA from the sample from the sample; identifying a large plurality of DNA and/or RNA variants from the sequenced data; identifying from the said large plurality of DNA and/or RNA variants those causing a qualitative difference in the corresponding encoded peptides and/or those causing a generation of a novel peptide sequence; and generating the said plurality of neoantigens, each neoantigen comprising one of the said identified difference in the encoded peptide.

## Description

### Technical domain

The present disclosure concerns analysis linked to genome in order to identify personalized neoantigens of a patient. In particular, the present disclosure concerns the discovery of a process for generating a plurality of neoantigens of a tumor of the patient.

More specifically, the present disclosure refers to a pool of DNA sequences encoding for a plurality of neoantigens.

In oncology, personalized vaccine, e.g. personalized therapeutic vaccine comprising tumor-specific antigens (neoantigens) of the tumor of the patient, to treat the tumor, hold great promise as a next-generation of personalized cancer immunotherapy.

### State of the art

The concept of personalized cancer vaccination is based on the identification of the neoantigens able to trigger the appropriate immunological response. This will allow to generate an optimal personalized vaccine, which will specifically boost the patient's immune system in attacking the tumor.

In that context, multiple methods, based on algorithm predictions, were developed to try to identify the neoantigens that will be the best candidates for personalized cancer vaccines.

Document EP3759131 provides an optimized approach for identifying and selecting neoantigens for personalized cancer vaccines, for T-cell therapy, or both. Optimized tumor exome and transcriptome analysis approaches for neoantigen candidate identification using next-generation sequencing (NGS) are addressed. These approaches include, depending on the embodiment, a trained statistical regression or nonlinear deep learning model that is configured to predict presentation of peptides of multiple lengths on a pan-allele basis. The model allows a more reliable prediction of the presentation of peptides and enables more time- and cost-effective identification of neoantigen-specific or tumor antigen-specific T- cells for personalized therapy using a clinically practical process that uses limited volumes of patient peripheral blood, screens few peptides per patient, and does not necessarily rely on MHC multimers. However, the predicting algorithms developed in this document still identify a significant number of false positive neoantigens that will not trigger a proper immunological response within the patient.

Document EP3813848 provides a computerized system for creating a nucleic acid cancer vaccine that has a maximized cancer efficacy for a given length. The document provides a cancer vaccine having a maximized anti-cancer efficacy for a given length and comprising one or more nucleic acids that can direct the body's cellular machinery to produce nearly any cancer protein or fragment thereof of interest. Maximized anti-cancer efficacy may be determined by identifying a T-cell activation value or survival value.

Currently all methods to identify a plurality of neoantigens that will be incorporated into a personalized vaccine rest, at least in part, from the predictions of algorithms that try to identify the best neoantigen candidates that will trigger an appropriate immunological response in the patient. Unfortunately, although these documents describe the merits of their technology, currently no method exists allowing the identification of neoantigens that will trigger an appropriate immunological response with a high success level. Indeed, despite the use of predicting algorithms, only a small fraction (4 to 20%) of the identified neoantigens will elicit an immunological response detectable by the apparition of T cells reacting to the neoantigens within the patient. The present inventors do consider that the methods known in the state of the art to identify the best neoantigen candidates are biased, at least because they are based on affinity data between the neoantigen peptide and the HLA receptors available only for particular types of HLA receptors. Thus, the current methods to identify the best neoantigen candidates might reach some efficacy for patient having said particular types of HLA receptors, while the methods will be inefficient for a large proportion of patients having different particular types of HLA receptors. On the other hand, the methods of the art represent in a certain way an optimum, since the use of the prediction algorithms is considered to result into an increased response.

### Summary of the invention

There is therefore a need to dispose of a process for identifying neoantigens from a sample of a tumor biopsy, wherein the neoantigens will have affinity to the HLA receptor types of the patient in order to trigger an appropriate T cell response. To achieve this, the inventors have developed a process to identify a plurality of neoantigens of the patient, wherein the plurality of neoantigens will provide an immunological response, when incorporated into a personalized vaccine into the patient, regardless of the type of HLA receptors of the patient and avoiding the pitfalls and biased results linked to the designed algorithms.

The present invention provides a process for generating a plurality of neoantigens from a sample obtained in a patient comprising the steps of:
- sequencing the DNA and/or the RNA from the sample;
- identifying a large plurality of DNA and/or RNA variants from the sequenced data;
- identifying from the said large plurality of DNA and/or RNA variants those causing at least one qualitative difference at the peptide level, for instance in the corresponding encoded peptides (mismatch mutation, frameshift, chromosomal reorganization, different RNA splicing) and/or those causing a generation of a novel peptide sequence encoded from an open reading frame wherein said open reading frame is only present in the tumor; and generating the said plurality of neoantigens, each neoantigen comprising the identified at least one difference at the peptide level, for instance (i) one of the said identified difference in the encoded peptide and amino acids upstream and/or downstream the said identified difference so as to form the neoantigen, or (ii) at least a part of the novel peptide sequence so as to form the neoantigen. Indeed, in the process according to the present invention, the plurality of neoantigens corresponds to the plurality of DNA variants causing (i) a qualitative difference in the corresponding encoded peptides or (ii) the generation of novel peptides, or the plurality of neoantigens is an selection of many (e.g. more than 50, more than 60, more than 100), if not all tumor neoantigens identified in the patient, which is independent from the type of HLA receptors of the patient. As a consequence, the process according to the present invention provides a plurality of neoantigens able to trigger, when incorporated into a personalized vaccine, an immunological response in the patient.

According to the present invention, the immunological response in the patient refers to the production of specific T cells targeting the neoantigens.

The present invention also relates to a pool of DNA and/or of RNA sequences encoding for said plurality of neoantigens obtainable by the process according to the present invention.

Other characteristics and advantages of the present invention will be derived from the non-limitative following description, and by making reference to the drawings and the examples.

The inventors developed a process for generating a plurality of neoantigens from a sample obtained in a patient comprising the steps of:
- sequencing the DNA from the sample;
- identifying a large plurality of DNA and/or the RNA variants from the sequenced data;
- identifying from the said large plurality of DNA and/or the RNA variants those causing a qualitative difference at the peptide level, for instance in the corresponding encoded peptides and/or those causing a generation of a novel peptide sequence encoded from an open reading frame wherein said open reading frame is only present in the tumor; and generating the said plurality of neoantigens, each neoantigen comprising (i) at least one of the said identified difference in the encoded peptide and amino acids upstream and/or downstream the said identified difference so as to form the neoantigen, or (ii) at least a part of the novel peptide sequence so as to form the neoantigen.

According to the present invention, the plurality of neoantigens corresponds to (i) the plurality of DNA variants causing a qualitative difference in the corresponding encoded peptides and/or causing a generation of a novel peptide sequence encoded from an open reading frame wherein said open reading frame is only present in the tumor, or (ii) to a selection from all tumor neoantigens identified in the patient, this selection being preferably independent from the type of HLA receptors of the patient.

According to the present invention the term "neoantigen" means a tumor-specific antigen of the patient, wherein the tumor-specific antigen is defined by an amino acid sequence having at least a qualitative difference as compared to the amino acid sequence of the corresponding encoded peptide, or wherein the tumor-specific antigen is defined by an amino acid sequence corresponding to a novel peptide sequence encoded from an open reading frame wherein said open reading frame is only present in the tumor and not in normal cells of the patient.

Preferably, the independent selection of a plurality of neoantigens is a (random) selection of at least 10%, preferably at least 20%, 30%, 40%, 50%, 60%, 70%, 80%, 90%, of all tumor neoantigens identified in the patient. Indeed, among such a plurality of neoantigens independently selected, based on a selection of all identified neoantigens in the patient, several neoantigens will have an affinity for the type of HLA receptors of the patient and will elicit an immunological response defined by the apparition of T cells responding to the neoantigens.

According to the present invention, the sample from the patient may originate from a solid and/or a liquid biopsy of the patient, preferably a solid biopsy.

According to the present invention, the sample comprises DNA and/or RNA originating from at least 1 tumor cell of the patient. Preferably, the sample further comprises DNA and/or RNA from a blood sample (for instance peripheral blood mononuclear cell (PBMC)) of the patient. More preferably, the sample comprises a first tumor sample comprising DNA and/or RNA originating from at least 1 tumor cell of the patient and a second normal sample comprising DNA and/or RNA from a blood sample of the patient and/or DNA and/or RNA from a matched normal tissue of the patient. Favorably, the first tumor sample comprises DNA and RNA originating from at least 1 tumor cell of the patient. The first tumor sample and the second normal sample are distinct samples. According to the present invention, a first tumor sample comprising DNA originating from at least 1 tumor cell of the patient and a first tumor sample comprising RNA originating from at least 1 tumor cell of the patient can be distinct samples. More preferably, the first tumor sample comprising DNA originating from at least 1 tumor cell of the patient and the first tumor sample comprising RNA originating from at least 1 tumor cell of the patient are the same sample. Sequencing the DNA can be performed by any methods of sequencing, such as high-throughput sequencing, pyrosequencing, sequencing-by-synthesis, single-molecule sequencing, nanopore sequencing, semiconductor sequencing, sequencing-by-ligation, sequencing-by-hybridization, RNA-Seq (Illumina), Digital Gene Expression (Helicos), Next-generation sequencing, Single Molecule Sequencing by Synthesis (SMSS) (Helicos), massively-parallel sequencing, Clonal Single Molecule Array (Solexa), shotgun sequencing, Maxam-Gilbert or Sanger sequencing, primer walking, sequencing using PacBio, SOLiD, Ion Torrent, or Nanopore platforms and any other sequencing methods known in the art. Preferably, sequencing the DNA is performed by a next-generation sequencing method. The next-generation sequencing method corresponds to a high-throughput, massively parallel, DNA sequencing approach. Sequencing the DNA is advantageously performed on a NextSeq500/550 or a NovaSeq sequencer or any Illumina or MGI sequencer.

The whole DNA, the exome or specific targeted DNA of the patient can be sequenced. Preferably, the whole DNA of the patient is sequenced. Preferably, the whole DNA of the first tumor sample and the second normal sample is sequenced. More preferably, the whole DNA of the first tumor sample and of the second normal sample and the RNA of the first tumor sample are sequenced.

Advantageously, sequencing the DNA is a single-cell DNA sequencing of at least 5 different tumor cells, preferably at least 20 different tumor cells, most preferably at least 100 different tumor cells, favorably at least 1000 different tumor cells of the patient. Indeed, depending on the type of the tumor and/or depending on the tumor of the patient, the tumor may be characterized by a high level of intra-tumoral heterogeneity, wherein the mutations in a tumor cell may differ from the mutations of another tumor cell. As a consequence, a single-cell DNA sequencing of the tumor will permit the identification of more neoantigens and can thus be very helpful in preventing clonal resistance towards a personalized vaccine encoding for a plurality of neoantigens of the patient. More preferably, the plurality of neoantigens identified originates from more than one tumor cell.

Advantageously, the process according to the present invention identifying a plurality of neoantigens is performed more than once, preferably at different stages of the tumor evolution and/or before and after a tumor treatment. Indeed, during tumor evolution, the mutations defining the tumor may evolve where some mutations may disappear while others may appear. As a consequence, the tumor may escape from the attack of specific T cells targeting neoantigens. Preferably, the plurality of neoantigens identified by the process according to the present invention corresponds to neoantigens identified in the most recent sample collected from the patient, wherein the sample comprises DNA originating from at least 1 tumor cell of the patient.

According to the present invention, sequencing the DNA from the sample comprises sequencing the DNA from the first tumor sample. Preferably, sequencing the DNA from the sample comprises sequencing the DNA from the first tumor sample and the DNA from the second normal sample.

According to the present invention, the DNA variant is identified by comparing the sequenced data from the first tumor sample with the sequenced data from the second normal sample.

According to an alternative version, in the present invention, identifying a large plurality of DNA variants from the sequenced data is performed by comparing the sequenced data with a reference genome. Preferably, the reference genome is a human genome. More preferably, the nucleotide sequence of the human genome is obtained from public database such as hg 19, i.e.: grch37, from NCBI. However, in order to determine a personalized DNA variant of the tumor of the patient, it is advantageous to compare the sequenced data from the tumor with the corresponding sequenced data from the normal tissue and/or normal cells, i.e. PBMC, of the patient.

Preferably, the DNA variant can be a DNA variant known to be often present in a particular tumor type of the patient and/or a personalized DNA variant identified in the tumor of the patient. The DNA variant can be a driver mutation or a "passenger mutation". Driver mutations are mutations that cause the initiation and/or the development of the tumor, while passenger mutations are mutations that accumulate during tumor evolution but show no direct role in the evolutionary process of the tumor. The DNA variant can be a point mutation and/or a single nucleotide polymorphism and/or a translocation and/or a frameshift and/or an indel, and/or a deletion, and/or a fusion (of two distinct nucleotide segments; i.e. a translocation event). The DNA variant can be present in an exonic and/or an intronic region and/or into silenced part of the sequenced DNA. According to the present invention, the DNA variant can be present in open reading frames, wherein said open reading frames are present in the DNA from the first tumor sample and in the DNA from the second normal sample. The DNA variant can also be present in new open reading frames, wherein said new open reading frames are only present in the first tumor sample, wherein said new open reading frames correspond to regions of the DNA of the second normal sample with no capacity of translation into a peptide (no promoter region, no initiation codon, premature STOP codon).

Preferably, sequencing the DNA and sequencing the RNA (in the first tumor sample) are performed on DNA and RNA coming from the same sample.

Preferably, the qualitative difference in the encoded peptide is determined by an identification of the open reading frames for which mRNAs are identified from RNA sequenced data from the tumor, wherein mRNAs have (i) a predicted peptide sequences having at least one different amino acid as compared to a reference peptide sequence and/or (ii) a de novo predicted peptide sequence. Preferably, the qualitative difference (under (i) here above) can be the result of a point mutation causing a different amino acid as compared to the reference peptide sequence or the result of a translocation, a deletion, an indel, and/or a fusion. The reference peptide sequence is preferably the predicted peptide sequence from the DNA sequenced data from the second normal sample of the patient.

Preferentially, the neoantigens comprise 2, 3, 4, 5, 6, 7, 8, 9, 10, 11, 12, 13amino acid(s) upstream and/or downstream the identified at least one difference, the said upstream amino acids being possibly identical in the tumor and in the non-tumor cells. The neoantigens are preferentially sequences of at least 7 amino acids in length, preferably more than 10 amino acids in length, more preferably more than 15 amino acids in length, even more preferably more than 20 amino acids in length, preferably less than 200 amino acids in length, more preferably less than 100 amino acids in length, even more preferably less than 50 amino acids in length, favorably around 27 amino acid in length. Preferentially, the qualitative difference is located substantially in the middle of the sequence of the neoantigen. Preferably, the neoantigens may also comprise at least a part of the novel peptide sequence encoded from an open reading frame wherein said open reading frame is only present in the tumor, wherein said at least a part of the novel peptide sequence is preferentially sequences of at least 7 amino acids in length, preferably more than 10 amino acids in length, more preferably more than 15 amino acids in length, even more preferably more than 20 amino acids in length, preferably less than 200 amino acids in length, more preferably less than 100 amino acids in length, even more preferably less than 50 amino acids in length, favorably around 27 amino acid in length.

Preferably, the neoantigen sequence is converted into a nucleic acid sequence by means of tools (i) optimizing the use of codon for an optimal antigen/neoantigen expression in human and/or (ii) reducing the formation of secondary structure of the nucleic acid sequence and/or (iii) avoiding RNA motives associated to an unwanted immune response and/or (iiii) favoring motives associated to a wanted immune response.

Preferably, the generated neoantigens represent at least 10% (at least 20, 30, 40, 50, 60, 70, 80, 90%, substantially all) of all the identified differences in the encoded peptides. Indeed, if the generated neoantigens represent a large percentage of the identified differences in the encoded peptide, it is more likely that, among the neoantigens, some of said generated neoantigens will elicit, when introduced into a personalized vaccine, a stronger immunological response in the patient and thus will increase the efficacy of the personalized vaccine.

Preferably, the identified plurality of the neoantigens according to the present invention is incorporated into a plurality of different constructs, preferably one construct per neoantigen.

Advantageously, each construct of the plurality of the different constructs is a synthetic DNA molecule comprising one segment encoding a tumor neoantigen under the control of a promoter for the transcription into a corresponding RNA molecule and a segment for the translation of the said transcribed RNA molecule into a peptide. Preferably, the synthetic DNA molecule further comprises a segment encoding a (peptide) sequence for stabilizing and/or targeting and/or trafficking the cell-synthetized tumor neoantigen in a vesicular region within the cell and/or at the cell surface. More preferably, the synthetic DNA molecule comprises a segment encoding a (peptide) sequence for addressing the encoded tumor neoantigen to MHC molecules and/or a segment encoding a translation enhancer and/or a 3'-UTR and/or a 3' Poly A tail segment(s) and/or a 5'-UTR. Preferably, the construct will serve as a DNA template for in vitro transcription to generate a RNA template that can be used in a personalized RNA vaccine of the patient.

Preferably, more than 20%, 30%, 40%, 50%, 60%, 70% 80%, 90% of the DNA of the patient is sequenced. Indeed, by increasing the sequencing percentage of the DNA of the patient, the plurality of DNA variants identified increases, allowing a comprehensive and/or more complete view on a personalized variants profile of the tumor of the patient, which permits the generation of a more complete view on the neoantigens profile of the tumor of the patient.

The present invention also relates to a pool of DNA sequences encoding said plurality of neoantigens, which is obtainable by the process according to the present invention.

Preferably, said plurality of neoantigens comprises at least 5, preferably at least 10, more preferably at least 40, even more preferably at least 100 different neoantigens. Indeed, to trigger a proper immunological response with a personalized vaccine comprising tumor neoantigens of the tumor of the patient, it is advantageous that the vaccine incorporates a large proportion of the neoantigens identified in the tumor of the patient. The number of tumor neoantigens can vary significantly from the type of the tumor, where some tumors are defined as 'cold tumors' with a very few number of mutations and of neoantigens while other tumors are defined as 'hot tumors' characterized by a very large number, e.g. more than 100, of different tumor neoantigens.

### Examples.-

### Example 1: Collect of DNA from a tumor sample and preparation of the DNA for sequencing

In a first step, the inventors have collected DNA from a tumor sample. To do so, the following steps have been performed:
- From a paraffin-embedded block comprising a tumor biopsy of the patient, cut the block into slides of 7 µm thick,
- stain the first and last slide with hematoxilin eosin (H&E slides),
- identify from the first slide, by visual inspection, of an area of the slide comprising sufficient tumor cells in good shape
- on this first colored slide, mark a region comprising the most tumor cells,
- transfer of the marking of the first slide on the unstained slides to mark the same region of the tumor, but on a different slice,
- crap cells from these marked regions (macrodissection step),
- extract the DNA from these slides to form the DNA for sequencing,
- in parallel, quantify the DNA,
- in parallel, qualify the extracted DNA.
The DNA can be used for sequencing.

### Example 2: Collect of RNA from a tumor sample and preparation of the RNA for sequencing.

To collect the RNA from a tumor sample, the inventors have performed the same steps as described in the Example 1 but they collect the RNA instead of the DNA.

Then, the RNA can be used for sequencing.

### Example 3: Sequencing the DNA from a sample isolated from the tumor and from the PBMC isolated from blood obtained from the patient.

From the DNA collected in the Example 1 and collected from the PBMC isolated from blood, a whole genome sequencing was carried out; a NovaSeq sequencer has been used here, using the NovaSeq S2 flow cells where the sequencing carried out is 'paired end sequencing': 2^{∗}150 bp. The average sequencing coverage is at least 50X. This sequencing made it possible to obtain a passage filter of the clusters of 90% for a total number. Sequencing readings of approximately 4,000,000,000 (4 billion / 8 samples).

### Example 4: Sequencing the RNA from a tumor sample obtained from the patient.

From the RNA collected in the Example 2, the inventors prepared libraries with NEBNext^{®} Ultra^{™} II Directional RNA Library Prep Kit for Illumina (NEB) according to supplier recommendations. The key steps of this protocol are : (i) the removal of ribosomal RNA fraction from 80 ng of total RNA using the QIAseq FastSelect -rRNA HMR Kits (Qiagen), (ii) a fragmentation using divalent cations under elevated temperature to obtain approximately 300 bp fragments, (iii) double strand cDNA synthesis, using reverse transcriptase and random primers, and (iv) Illumina adapters ligation and cDNA library amplification by PCR for sequencing. RNA Sequencing is then carried out in paired-end 100b mode with an Ilumina NovaSeq 6000.

### Example 5: Identification of DNA variants from the tumor of the patient.

Using bioinformatic analysis, e.g. ABRA (https://academic.oup.com/bioinformatics/article/30/19/2813/2422200), SAMTOOLS (https://www.ncbi.nlm.nih.gov/pmc/articles/PMC2723002/), BEDTOOLS (https://academic.oup.com/bioinformatics/article/26/6/841/244688 ), or FASTP (https://academic.oup.com/bioinformatics/article/34/17/i884/5093234), the sequenced data obtained by the whole genome sequencing (see Example 3) have allowed to identify the plurality of DNA variants of the tumor of the patient by comparing the sequenced data of the tumor and the sequenced data of the PBMC.

### Example 6: Identification of neoantigens of the tumor of the patient.

The open reading frames for which mRNAs are detected from the RNA sequencing (see Example 4) have been identified. Then, the mRNAs having a predicted peptide sequence having at least one different amino acid as compared to the predicted peptide sequence coming from the DNA sequenced data from the PBMC have been selected. The inventors have then checked if the selected mRNAs originate from the DNA variants identified in the Example 5.

Then the inventors have identified the neoantigens of the tumor by predicting the peptide sequences of the selected mRNAs.

### Example 7: Generation of a plurality of neoantigens from the tumor of the patient.

The inventors have identified the difference in the predicted peptide sequences of the selected mRNAs (see Example 6) with the corresponding predicted peptide sequences coming from the DNA sequenced data from the PBMC.

On that basis, 20 neoantigens of 27 amino acids, having each a different amino acid sequence as compared to the corresponding sequence of the matched normal tissue have been designed. For each neoantigen, the difference is located substantially in the middle of the amino acid sequence of 27 amino acids. These 20 neoantigens have amino acid sequences corresponding to SEQ ID NO :1 to SEQ ID NO :20. In addition, 40 random epitopes (antigens) have been designed as internal control for in vitro testing having the amino acid sequences corresponding to SEQ ID NO :21 to SEQ ID NO :60.

## Claims

1. A process for generating a plurality of neoantigens from a sample obtained from a patient comprising the steps of:
- sequencing the DNA and/or the RNA from the sample;
- identifying a large plurality of DNA and/or RNA variants from the sequenced data;
- identifying from the said large plurality of DNA and/or RNA variants those causing a qualitative difference at the peptide level; and generating the said plurality of neoantigens, each neoantigen comprising at least one of the said identified difference in the encoded peptide and amino acids upstream and/or downstream the said at least one identified difference so as to form the neoantigen.

2. The process according to claim 1, wherein the sample comprises a first tumor sample comprising DNA and/or RNA originating from at least 1 tumor cell of the patient and a second normal sample comprising DNA and/or RNA from a blood sample of the patient and/or DNA and/or RNA from a matched normal tissue of the patient.

3. The process according to claim 1 or 2, wherein the DNA variant is identified by comparing the sequenced data from the first tumor sample with the sequenced data from the second normal sample.

4. The process according to any one of the preceding claims, wherein more than 20%, 30%, 40%, 50%, 60%, 70% 80%, 90% of the DNA of the patient is sequenced.

5. The process according to any one of the preceding claims, wherein the generated neoantigens represent at least 10% (at least 20, 30, 40, 50, 60, 70, 80, 90%, substantially all) of all the identified differences in the encoded peptides.

6. The process according to any one of the preceding claims, wherein the identified plurality of the neoantigens is incorporated into a plurality of different constructs, preferably one construct per neoantigen.

7. The process according to any one of the preceding claims, wherein the neoantigens comprise 2, 3, 4, 5, 6, 7, 8, 9, 10, 11, 12, 13 amino acid(s) upstream and/or downstream the identified at least one difference, the said upstream amino acids being possibly identical in the tumor and in the non-tumor cells.

8. The process according to any one of the preceding claims, wherein the qualitative difference in the encoded peptide is determined by an identification of the open reading frames for which mRNAs are identified from the first tumor sample, wherein mRNAs have (i) a predicted peptide sequences having at least one different amino acid as compared to a reference peptide sequence and/or (ii) a de novo predicted peptide sequence.

9. The process according to any one of the preceding claims, wherein the whole DNA of the patient is sequenced.

10. A pool of DNA or of RNA sequences encoding for said plurality of neoantigens obtainable by the process according to any one of the preceding claims.

11. The pool according to claim 10, wherein said plurality of neoantigens comprises at least 5, preferably at least 10, more preferably at least 40, even more preferably at least 100 different neoantigens.
